# EUROPÄISCHE PATENTANMELDUNG

(11) **EP 2 921 099 A1**
(43) Veröffentlichungstag der Anmeldung: **23.09.2015**
(21) Anmeldenummer: 14160520.4
(22) Anmeldetag: 18.03.2014
(51) Int. Cl.: A61B 3/13, G02B 21/00, G02B 25/00, A61B 3/12

(54) **Ophthalmoskopie-Vorsatzeinheit für Operationsmikroskop**

(71) Anmelder: Dieter Mann GmbH, 63814 Mainaschaff (DE)
(72) Erfinder: Mann, Dieter, 63839 Kleinwallstadt (DE)
(74) Vertreter: Prüfer & Partner GbR European Patent Attorneys

(57) **Zusammenfassung**

Eine Ophthalmoskopie-Vorsatzeinheit für ein Operationsmikroskop wird zwischen der Frontlinse (1b) des Operationsmikroskops und einem Patientenauge positioniert und weist eine indirekte Linse (8a) zur Erzeugung eines reellen Bildes (Z) des Augenhintergrunds sowie eine Anpassoptik (9) auf. Die Anpassoptik ist zwischen der Frontlinse (1b) und der indirekten Linse (8a) angeordnet und dient der Anpassung des Wegs des Lichts vom reellen Bild (Z) zur Frontlinse (1b). Für die Anpassung des Lichtwegs wird eine Spiegelanordnung (91, 92, 93, 94) verwendet.

## Beschreibung

Seit Anfang der Achtzigerjahre des vergangenen Jahrhunderts sind Ophthalmoskopie-Zusätze zu einem Operationsmikroskop bekannt, mit denen während vitreoretinaler Eingriffe (Glaskörper- und Netzhaut-Operationen) eine Weitwinkelabbildung der Retina erzeugt wird. Diese Zusätze basieren auf dem 1861 von Giraud-Teulon angegebenen Prinzip der indirekten Ophthalmoskopie. Mit einer Linse, die vor dem Auge positioniert ist, erzeugt man ein indirektes (umgekehrtes, auf dem Kopf stehendes und seitenverkehrtes) reelles (Zwischen-)Bild vom Augenhintergrund (Fundus), das mit dem OP-Mikroskop beidäugig betrachtet wird. Solch eine unmittelbar vor dem Auge positionierte Linse wird in der vorliegenden Beschreibung auch als Ophthalmoskopierlinse, indirekte Linse oder Vorsatzlinse bezeichnet.

Sowohl in EP 1 227 355 A2 als auch in EP 2 316 330 B1 werden die Inzisionen (Öffnungen am äußeren Auge, also am Vorderabschnitt des Auges, für das Eingehen mit den chirurgischen Instrumenten) mit dem OP-Mikroskops ohne in den Strahlengang eingebrachten Ophthalmoskopie-Zusatz betrachtet, während die Netzhaut über den in den Strahlengang eingeschwenkten Ophthalmoskopie-Zusatz betrachtet wird. Die Netzhaut wird dabei über das durch die vor dem Auge positionierte Linse erzeugte reelle Bild betrachtet. Dieses befindet sich zwischen Ophthalmoskopierlinse und Mikroskopobjektiv, so dass die zugehörige Scharfeinstellung des Mikroskops unterschiedlich zur Scharfeinstellung für die Stelle der Inzisionen ist.

Für die Bildumkehr des indirekten reellen Bildes in ein aufrechtes seitenrichtiges und nicht mehr auf dem Kopf stehendes Bild wurden 1958 von Donaldson und 1967 von Kurt Schirmer Prismen vorgeschlagen. Solche Prismen, vorzugsweise ein Schmidt/Pechan-Prisma oder ein Uppendahl-Prisma, werden in sogenannten separaten Invertern, wie sie in EP 1 227 355 A2 oder EP 2 316 330 B1 beschrieben sind, verwendet. In EP 2 316 330 B1 ist dabei die aus drei Prismen bestehende Prismenanordnung mit der indirekten Linse zusammen in ein gemeinsames Gehäuse eingebaut. Die in EP 1 227 355 A2 und EP 2 316 330 B1 verwendeten Prismenanordnungen haben gleichzeitig den Vorteil, dass der Lichtweg verlängert wird und dadurch für die Betrachtung des reellen Zwischenbildes des Fundus und die Betrachtung des Vorderabschnitts des Auges ein und dieselbe Scharfeinstellung des Mikrokops verwendet werden kann.

Sowohl in EP 1 227 355 A2 als auch in EP 2 316 330 B1 wird eine Einstellung auf eine neue Schärfenebene (beispielsweise tieferliegende Bereiche des Glaskörpers) über eine Vertikalverstellung des OP-Mikroskops vorgenommen. In beiden Fällen ist der Mikroskopzusatz mit der Ophthalmoskopierlinse (indirekten Linse) an der Unterseite des OP-Mikroskops befestigt und dadurch an das OP-Mikroskop gekoppelt. Diese Kopplung ist nachteilig, wenn z.B. das Auge sich bewegt oder im Verlaufe eines Eingriffs tieferliegende Bereiche des Glaskörpers betrachtet werden sollen. Da in diesen Fällen das Objektivs auf eine neue Schärfenebene eingestellt werden muss, ändert sich durch die Kopplung auch der Abstand der indirekten Linse zum Auge. Dies wiederum führt aber zu einer Unschärfe des reellen Bildes des Augenhintergrunds, was eine Nachfokussierung erforderlich macht.

In EP 1 227 355 A2 ist daher zur Erleichterung der Fokussierung eine Reduzierlinse von ca. +2,0 Dioptrien zwischen Mikroskopobjektiv und indirekter Linse vorhanden.

In EP 2 316 330 B1 ist das mittlere der drei Prismen ein Bauernfeindprisma mit Dachkante, welches zur Veränderung der Länge des Strahlengangs verschiebbar ist. Damit ist ebenfalls eine Scharfeinstellung für das Zwischenbild vom Fundus möglich.

Die Verwendung von Prismenanordnungen führt einerseits zu höheren Kosten und andererseits zu einem Helligkeitsverlust. Es ist daher eine Aufgabe der vorliegenden Erfindung, eine Ophthalmoskopie-Vorsatzeinheit für ein Operationsmikroskop bereitzustellen, welche kostengünstig und lichtstark ist.

Die Aufgabe wird gelöst durch eine Ophthalmoskopie-Vorsatzeinheit nach Anspruch 1. Weiterbildungen der Erfindung sind in den Unteransprüchen angegeben.

Nachfolgend werden Ausführungsbeispiele der vorliegenden Erfindung unter Zuhilfenahme der beigefügten Zeichnungen beschrieben. Von den Figuren zeigen:
- Fig. 1: die in den Strahlengang zwischen Augenhintergrund und Mikroskopobjektiv eingefügte Ophthalmoskopie-Vorsatzeinheit gemäß der vorliegenden Erfindung,
- Fig. 2: eine Seitenansicht der in Fig. 1 dargestellten Ophthalmoskopie-Vorsatzeinheit und
- Fig. 3: ein Operationsmikroskop mit der angebrachten erfindungsgemäßen Ophthalmoskopie-Vorsatzeinheit.

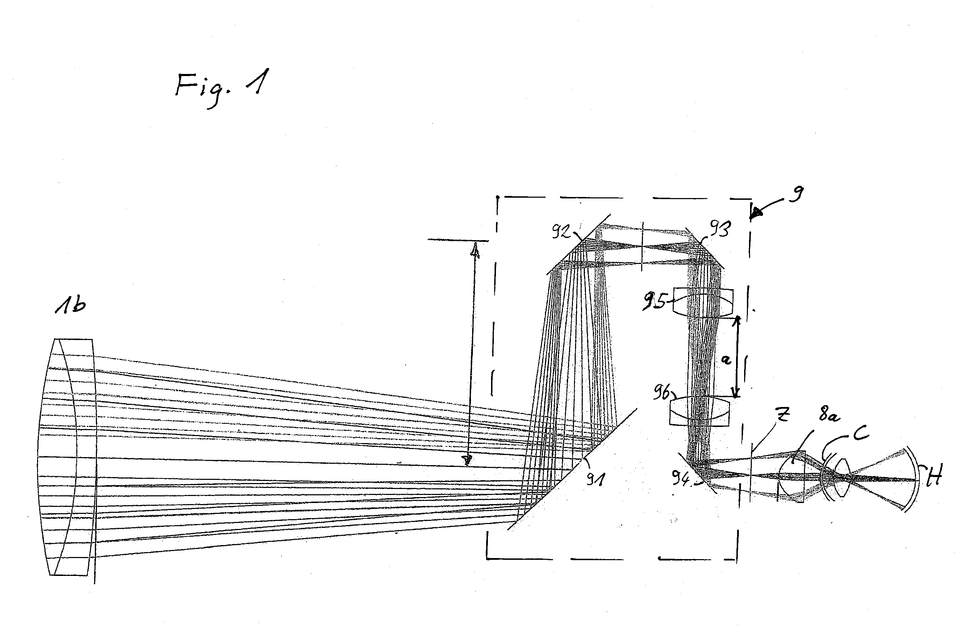

Fig. 1 zeigt schematisch eine Ausführungsform einer erfindungsgemäßen Ophthalmoskopie-Vorsatzeinheit mit eingezeichnetem Strahlengang bei Verwendung an einem Operationsmikroskop. Die Ophthalmoskopie-Vorsatzeinheit ist räumlich zwischen der Objektivlinse (Frontlinse) 1b des Mikroskops und dem Patientenauge angeordnet. Die Vorsatzeinheit weist eine Anpassoptik 9 und eine indirekte Linse 8a auf, welche zwischen der Cornea eines Patientenauges und der Anpassoptik 9 positioniert ist, so dass durch die indirekte Linse 8a ein reelles Zwischenbild Z des Augenhintergrunds zwischen indirekter Linse 8a und Anpassoptik 9 erzeugt wird. Bei der indirekten Linse 8a handelt es sich bevorzugt um eine einzelne Linse, auch als Ophthalmoskopierlinse bezeichnet.

In der Figur 1 treten Lichtstrahlen vom Augenhintergrund in die Anpassoptik 9 auf deren rechter Seite ein und verlassen diese auf deren linker Seite, um zur Objektivlinse (Frontlinse) 1b zu gelangen. Die Anpassoptik 9 enthält vier Planspiegel 91, 92, 93, 94, welche das Licht so ablenken, dass sich die Länge des Beobachtungsstrahlengangs verlängert. Hierdurch ist es möglich, die Länge des Beobachtungsstrahlengangs so abzuändern, dass bei Vorhandensein der indirekten Linse 8a im Strahlengang ohne weitere Veränderungen am Mikroskop das Zwischenbild Z scharf gesehen werden kann.

Wie in der Figur zu sehen ist, erfolgt durch die Spiegel jeweils eine Ablenkung des Lichts um ca. 90°, so dass die parallel zur optischen Achse in die Anpassoptik 9 eintretenden Lichtstrahlen die Anpassoptik 9 wieder parallel zur optischen Achse verlassen. Das Licht nimmt in der also in der gezeigten Anpassoptik 9 einen U-förmigen Weg, wobei die eintrittsseitigen beiden Spiegel 93, 94 im Wesentlichen parallel zueinander sind, die austrittsseitigen Spiegel 91, 92 im Wesentlichen parallel zueinander sind und die eintrittsseitigen Spiegel im Wesentlichen senkrecht zu den austrittsseitigen Spiegeln angeordnet sind. Natürlich ist es auch möglich, eine größere gerade Zahl an Spiegeln als vier und/oder andere Ablenkwinkel zu wählen. Der optische Aufbau wird hierdurch jedoch komplizierter.

Durch die Verwendung von Spiegeln ist die Vorsatzeinheit kostengünstiger als bei Verwendung von Prismen. Insbesondere werden Lichtverluste durch den Durchtritt des Lichtes durch das Prismenmaterial vermieden.

Wie bereits in der Einleitung erwähnt wurde, erzeugt die indirekte Linse 8a ein auf dem Kopf stehendes und seitenverkehrtes Bild. Der Versuch, eine Höhen- und Seitenumkehr des Bildes allein mittels Spiegeln zu erreichen, führt zwangsläufig zu einem komplizierten Aufbau. Im Gegensatz zum bekannten Stand der Technik wird bei der vorliegenden Erfindung daher eine Bildaufrichtung und Seitenvertauschung nicht mittels Spiegeln oder Prismen bewerkstelligt, sondern mittels zweier Linsenelemente 95 und 96, die in Fig. 1 zwischen dem im Strahlengang der Vorsatzoptik 8a nächstgelegenen Spiegel 94 und dem zu diesem im Strahlengang nachfolgenden Spiegel 93 angeordnet sind. Im Gegensatz zu dem im Stand der Technik für eine Bildumkehr verwendeten Prismen und Spiegeln sind die Linsenelemente 95 und 96 aufgrund ihrer Radialsymmetrie ideal für diesen Zweck geeignet.

Für jedes der Linsenelemente 95 und 96 wird vorzugsweise ein Achromat verwendet, es ist jedoch auch durchaus möglich, jeweils eine einzelne Linse oder ein Linsensystem bestehend aus mehreren Linsen zu verwenden. Weiterhin ist es auch möglich, die Linsenelemente an anderen Stellen zwischen den vorhandenen Ablenkspiegeln im Aufbau anzuordnen.

Das Vorhandensein der beiden Linsenelemente 95 und 96 bringt als weiteren Vorteil mit sich, dass eine Scharfstellung auf das Zwischenbild Z durch Verfahren eines der beider Linsenelemente 95, 96 relativ zum anderen parallel zum Strahlengang bewirkt werden kann. Wenn beispielsweise bei dem Auge, an dem der Eingriff vorgenommen wird, eine Fehlsichtigkeit vorliegt, so wird infolge der veränderten Augenlänge (Abstand zwischen Cornea C und Hintergrund H) das Zwischenbild Z an einer anderen Stelle liegen. Durch das Verfahren von einem der Linsenelemente 95, 96 gegenüber dem anderen Linsenelement kann diese veränderte Lage des Zwischenbildes kompensiert werden, so dass das Zwischenbild Z auch an veränderter Position zusätzlich zu den Inzisionen scharf gesehen wird.

Die Praxistauglichkeit der soeben beschriebenen Anpassoptik 9 kann durch die im Folgenden beschriebenen Abwandlungen, welche einzeln oder in Kombination vorgenommen werden können, noch zusätzlich verbessert werden:

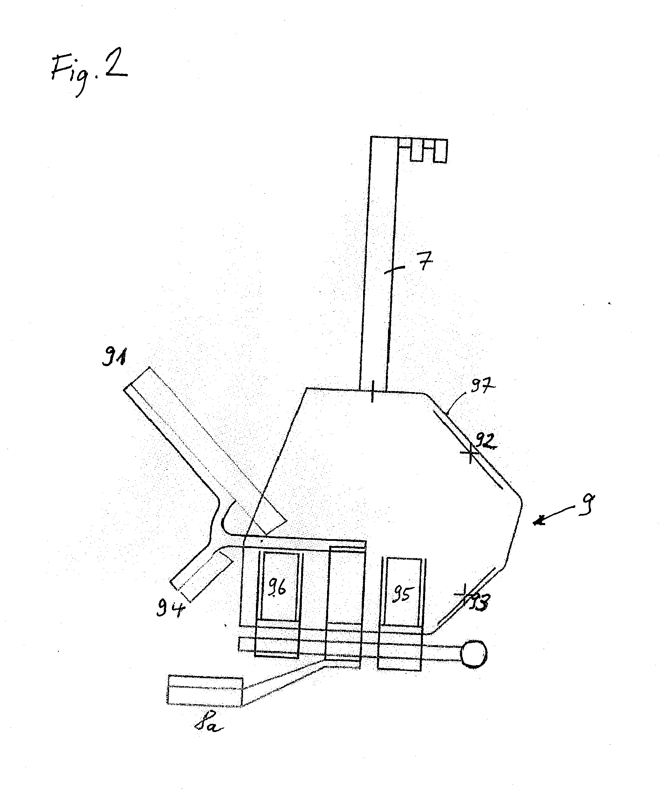

Wie in Fig. 2 dargestellt ist, kann beispielsweise die gesamte Anpassoptik 9 oder aber auch lediglich ein Teil derselben mit einer lichtdichten Umhüllung 97 versehen werden, die bevorzugt abnehmbar ist. Dadurch wird die Anpassoptik gegen Umgebungsstreulicht abgeschirmt, das sich störend auswirkt.

Die Spiegel sind bevorzugt mit einer Oberflächenverspiegelung versehen, die gegen die scharfen Reiniger bei einem Desinfektionsvorgang resistent ist, so dass die Anpassoptik 9 autoklavierbar ist, also insbesondere gegen Reiniger mit einem pH-Wert bis zu 11 und eine Behandlung unter Dampf bei 134°C und 2,4 bar mit einer Haltezeit von 15 Minuten resistent ist. Bevorzugt ist auch das vom RKI-Institut für die Sterilisation empfohlene Prionen-programm ohne Beschädigung der Spiegeloberflächen möglich. Eine Autoklavierbarkeit wird insbesondere dann erreicht, wenn die Oberflächen der Spiegel mit Rhodium beschichtet sind. Eine Rhodiumbeschichtung hat ferner den Vorteil, dass sie hervorragende Reflexionseigenschaften aufweist.

Weiterhin sind bevorzugt die Spiegel 91 bis 94 und die beiden Linsenelemente 95 und 96 in Steckfassungen gehaltert, so dass im Falle einer mechanischen Beschädigung ein einfacher Austausch möglich ist. Insbesondere im Gegensatz zu Prismenanordnungen des Standes der Technik können daher bei der erfindungsgemäßen Anpassoptik 9 auf einfache Weise Einzelelemente des optischen Systems ausgetauscht werden.

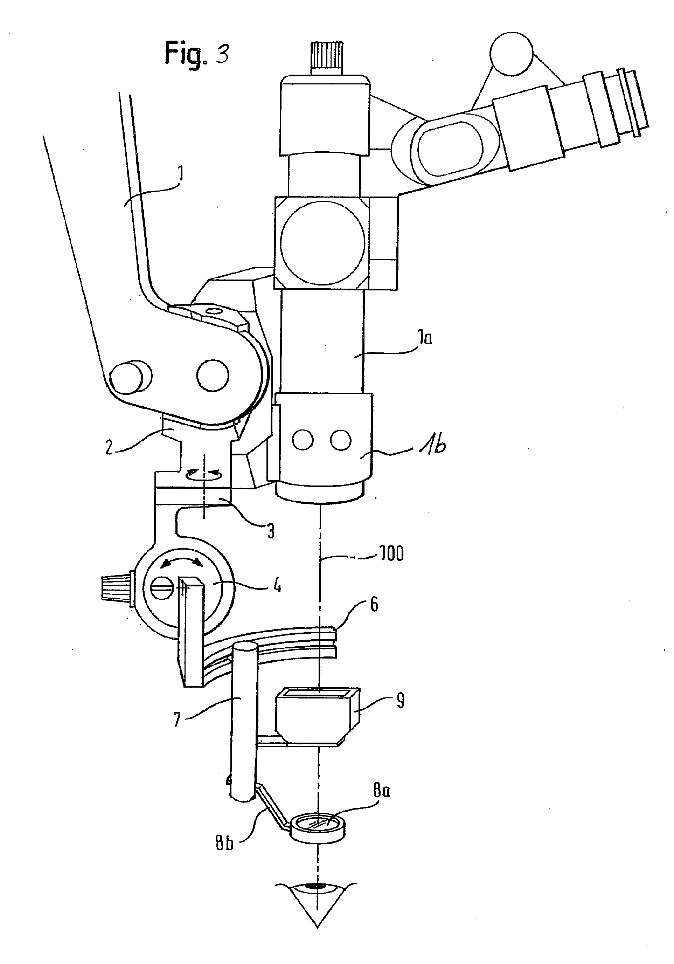

In Fig. 3 ist ein Operationsmikroskop, mit dem die erfindungsgemäße Ophthalmoskopie-Vorsatzeinheit bevorzugt eingesetzt werden kann, in schematischer Weise dargestellt. Insbesondere die Anpassoptik ist nur schematisch dargestellt. Der Aufbau ist an den in EP 2 244 118 B1 offenbarten Aufbau angelehnt.
Das Mikroskop ist in Fig. 3 mit einem Tubus 1a und einem daran befestigten Objektiv 1b mittels eines Haltearms 1 an einem nicht gezeigten Stativ angebracht. Insbesondere kann der Tubus 1a mit dem Objektiv 1b für eine Scharfstellung relativ zu dem Haltearm 1 in der Höhe verfahren werden.

Weiterhin erkennt man in Fig. 3, dass die Ophthalmoskopie-Vorsatzeinheit 8a, 8b, 9 über eine mehrteilige Befestigungsvorrichtung 2, 3, 4, 6, 7 an dem Haltearm 1 befestigt ist, so dass die Ophthalmoskopie-Vorsatzeinheit beim Verfahren des Mikroskopobjektivs 1b nicht mitbewegt wird. Falls die Befestigung über zwei Schwenkvorrichtungen 3 und 4 erfolgt, gestattet dies ein Verschwenken sowohl um eine horizontale Achse als auch um die optische Achse des Objektivs oder eine zu dieser parallele Achse. Optional ist noch mittels eines Knopfes 19 eine Verkippung um eine Achse 21 unmittelbar am Anbringungspunkt am Haltearm 1 des Mikroskops möglich.

Mittels einer Stabhalterung 7, welche in einer teilkreisförmigen (z.B. halbkreisförmigen) Schiene 6 verschiebbar ist, kann die Ophthalmoskopie-Vorsatzeinheit um die optische Achse des Objektivs gedreht werden. Damit ist eine problemlose Verdrehmöglichkeit beispielsweise beim Wechsel von einem Auge auf das andere gegeben.

Die Ophthalmoskopierlinse 8a ist in einem Ring montiert, welcher über einen Tragesteg 8b mit der Stabhalterung 7 verbunden ist. Durch die Stabhalterung 7 und den Tragesteg 8b ist daher die Ophthalmoskopierlinse 8a starr mit der Anpassoptik 9 verbunden. Die Stabhalterung 7 ist bevorzugt so in der Schiene 6 angebracht, dass sie bei einer nach oben in Richtung des Objektivs 1b wirkenden Kraft aus der Schiene ausgehangen wird. Dadurch wird eine Verletzung des Auges vermieden, falls bei einer versehentlichen Bewegung des Mikroskops relativ zum Haltearm 1 die Ophthalmoskopierlinse 8a das Auge berührt. Wird die Ophthalmoskopie-Vorsatzeinheit nicht benötigt, so kann sie durch Aushängen der Stabhalterung 7 aus der Schiene 6 aus dem Strahlengang entfernt. Die dann noch unter der Frontlinse 1 des Mikroskops zurückbleibende Schiene 6 kann dann optional mittels der Schwenkvorrichtungen 3 oder 4 aus dem Beobachtungsstrahlengang des Mikroskops geschwenkt werden. Da die Ophthalmoskopie-Vorsatzeinheit am Haltearm 1 des Mikroskops befestigt ist und Bewegungen des Mikroskopobjektivs 1b nicht mitmacht, muss beim Einschwenken in den Beobachtungsstrahlengang nicht eine komplizierte Justage zur Scharfstellung erfolgen, da der Abstand zwischen Ophthalmoskopierlinse 8a zum Auge bei Veränderungen an der Lage des Mikroskopobjektivs 1b nicht verändert wird.

Die erfindungsgemäße Ophthalmoskopie-Vorsatzeinheit ist gerade bei Verwendung in dem in Fig. 3 gezeigten Aufbau, in dem eine Anbringung am Haltearm 1 des Mikroskops vorgesehen ist, von Vorteil. Bei Veränderungen der Schärfenebene (beispielsweise bei einer fortschreitenden Operation am Glaskörper) muss, nachdem ohne Vorhandensein der Vorsatzeinheit eine Scharfeinstellung vorgenommen wurde, nach dem Einsetzen der Vorsatzeinheit, z.B. durch ein Einhängen der Stabhalterung 7 in die Schiene 6, für eine Scharfstellung des Fundusbildes allenfalls eine Justierung der Lage eines der beiden Linsenelemente 95, 96 vorgenommen werden.

Natürlich kann die erfindungsgemäße Ophthalmoskopie-Vorsatzeinheit statt am Haltearm 1 auch am Mikroskopobjektiv 1b angebracht werden, obwohl man dann den soeben beschriebenen Vorteil verliert. Für die Anbringung kann wiederum eine Befestigungsvorrichtung gewählt werden, die die oben beschriebene Stabhalterung 7 in der Schiene 6 verwendet, die Kombination aus Anpassoptik 9 und Ophthalmoskopierlinse 8a kann aber auch in anderer Weise am Mikroskop befestigt werden.

## Patentansprüche

1. Ophthalmoskopie-Vorsatzeinheit für ein Operationsmikroskop zur Positionierung zwischen der Frontlinse (1b) des Operationsmikroskops und einem Patientenauge mit
einer indirekten Linse (8a) zur Erzeugung eines reellen Bildes (Z) des Augenhintergrunds,
einer Anpassoptik (9), die zwischen der Frontlinse (1b) und der indirekten Linse (8a) angeordnet ist, wobei durch die Anpassoptik der Weg des Lichts vom reellen Bild (Z) zur Frontlinse (1b) angepasst wird,
**dadurch gekennzeichnet, dass** der Lichtweg durch die Anpassoptik (9) mittels einer Spiegelanordnung (91, 92, 93, 94) angepasst wird.

2. Ophthalmoskopie-Vorsatzeinheit nach Anspruch 1, wobei die Anpassoptik weiterhin mindestens zwei hintereinander im Strahlengang angeordnete Linsenelemente (95, 96) zur Aufrichtung und Seitenvertauschung des reellen Bildes (Z) aufweist.

3. Ophthalmoskopie-Vorsatzeinheit nach Anspruch 2, wobei der Abstand zwischen den Linsenelementen (95, 96) veränderbar ist.

4. Ophthalmoskopie-Vorsatzeinheit nach Anspruch 2 oder 3, wobei es sich bei den Linsenelementen um Achromate handelt.

5. Ophthalmoskopie-Vorsatzeinheit nach einem der Ansprüche 1 bis 4, wobei die Spiegel und/oder Linsenelemente der Anpassoptik mittels Steckfassungen gehaltert sind.

6. Ophthalmoskopie-Vorsatzeinheit nach einem der Ansprüche 1 bis 5, wobei die Spiegel autoklavierbare Spiegeloberflächen aufweisen.

7. Ophthalmoskopie-Vorsatzeinheit nach Anspruch 6, wobei die Spiegeloberflächen mit Rhodium beschichtet sind.

8. Ophthalmoskopie-Vorsatzeinheit nach einem der Ansprüche 1 bis 7, wobei die Anpassoptik ganz oder teilweise von einer abnehmbaren Umhüllung (97) aus lichtdichtem Material umgeben ist.

9. Ophthalmoskopie-Vorsatzeinheit nach einem der Ansprüche 1 bis 8, wobei die Spiegelanordnung aus vier im Strahlengang hintereinander angeordneten Spiegeln besteht, so dass ein U-förmiger Strahlengang entsteht.

10. Ophthalmoskopie-Vorsatzeinheit nach einem der Ansprüche 1 bis 9, wobei weiterhin eine Befestigungsvorrichtung (2, 3, 4, 6, 7) vorhanden ist, die so ausgelegt ist, dass die Ophthalmoskopie-Vorsatzeinheit mittels der Befestigungsvorrichtung an einem Haltearm (1) des Operationsmikroskops angebracht werden kann.
